# FASCICULE DE BREVET EUROPEEN

(11) **EP 2 726 029 B1**
(45) Date de publication et mention de la délivrance du brevet: **12.07.2017**
(21) Numéro de dépôt: 12733656.8
(22) Date de dépôt: 29.06.2012
(51) Int. Cl.: A61F 5/01, A61F 5/058

(54) **DISPOSITIF DE MAINTIEN D'UNE ARTICULATION D'UN UTILISATEUR**
VORRICHTUNG ZUR UNTERSTÜTZUNG EINES GELENKS EINES BENUTZERS
DEVICE FOR SUPPORTING A JOINT OF A USER

(30) Priorité: 01.07.2011 FR 1155958
(43) Date de publication de la demande: 07.05.2014
(73) Titulaire: Thuasne, 92300 Levallois Perret (FR)
(72) Inventeur: MARTIN, Frédéric, F-42100 Saint Etienne (FR); DE MONCUIT, Henri, F-42000 Saint Etienne (FR); CONVERT, Reynald, F-42800 Saint Martin La Plaine (FR)
(74) Mandataire: Lavoix
(86) Numéro de dépôt international: PCT/EP2012/062755
(87) Numéro de publication internationale: WO 2013/004633

(56) Documents cités:
- WO-A1-97/24085
- US-A- 5 695 453
- US-A1- 2008 287 848
- US-A1- 2009 012 438
- US-A1- 2011 046 529

## Description

La présente invention concerne un dispositif de maintien d'une articulation d'un utilisateur reliant une première partie du corps dudit utilisateur à une deuxième partie du corps dudit utilisateur, du type comprenant une première face et une deuxième face reliées l'une à l'autre par au moins un manchon, destiné à entourer la première partie du corps de l'utilisateur, et par une partie d'appui, destinée à être en appui contre la deuxième partie du corps dudit utilisateur, ledit manchon et ladite partie d'appui étant reliés l'un à l'autre par des moyens de blocage de la fermeture de l'articulation. L'invention s'applique plus particulièrement à des dispositifs médicaux de maintien, par exemple du type orthèse de poignet adaptée pour le traitement de rhizarthrose, ou arthrose de la base du pouce, ou arthrose périscaphoïdienne ou autres douleurs de la base du pouce.

Une telle orthèse est par exemple décrite dans les documents FR-2 854 564, FR-2 948 016 ou US 2009/0012438. Ces orthèses comprennent des moyens de blocage de la fermeture de l'articulation entre le pouce et l'index de sorte à empêcher la fermeture de la première commissure de la main. De tels moyens de blocage sont formés par un élément de blocage comprenant une partie d'appui contre la base du pouce et une partie d'appui contre la base de l'index et présentant une forme sensiblement en U ou en selle de cheval, c'est-à-dire que les parties d'appui sont reliées l'une à l'autre par une zone de jonction recouvrant la première commissure. Un manchon ou une sangle passent par la première commissure et recouvre l'élément de blocage afin de maintenir les parties d'appui en place, c'est-à-dire en appui contre la base du pouce et de l'index, ce qui permet d'empêcher la fermeture de la première commissure de la main lorsque l'orthèse est portée.

Cependant, même s'il peut être légèrement élastique, l'élément de blocage, par exemple réalisé en matériau plastique ou en métal, est sensiblement rigide et le manchon ou la sangle exerce un appui sur la zone de jonction. Ainsi, du fait de sa rigidité, l'élément de blocage exerce une contrainte sur la première commissure de la main, ce qui peut devenir douloureux pour le porteur de l'orthèse, en particulier lorsque celle-ci est portée fréquemment ou de façon prolongée.

Ainsi, l'orthèse, censée atténuer les douleurs et avoir un effet antalgique, peut occasionner un inconfort et de nouvelles douleurs à son porteur, du fait de la pression exercée par l'élément de blocage sur la première commissure de la main et donc sur l'articulation entre le pouce et l'index. Cet inconfort peut pousser le patient à retirer l'orthèse et donc à mal suivre le traitement, ce qui entraîne un ralentissement de l'amélioration de l'état de santé du patient.

De manière plus générale, tout dispositif de maintien d'une articulation devrait éviter d'exercer une contrainte sur cette articulation afin de permettre le port de ce dispositif de façon fréquente et/ou prolongée.

Le document US 5 695 453 décrit un dispositif d'immobilisation d'un membre comprenant un élément de corps en mousse dans lequel est compris un système d'éléments filaires de renforcement et de stabilisation. L'élément de corps est prêt à être facilement conformé à la forme et la taille d'un membre. Des ouvertures de l'élément de corps facilitent l'application de l'élément de corps à un membre.

Le document US 2008/0287848, qui représente l'état de l'art le plus proche, décrit une autre orthèse de poignet comportant des moyens de blocage de la fermeture de l'articulation entre le pouce et l'index formés par un élément filaire. Cet élément filaire comprend deux branches courbes qui s'étendent respectivement sur chacune des faces du dispositif et dont les extrémités distales sont reliées l'une à l'autre, au niveau du pouce, par une zone de jonction. Cette zone de jonction est fixée au pouce par une sangle tandis que les extrémités proximales des branches sont insérées dans des goussets formés sur un manchon entourant le poignet et dans lesquels elles peuvent coulisser. Ainsi, l'élément filaire n'exerce pas d'appui sur la première commissure tout en en empêchant la fermeture. L'un des buts de l'invention est de permettre le port d'un dispositif de maintien d'une articulation de façon confortable, même lorsque ce dispositif est porté de façon répétée ou pendant de longues périodes.

A cet effet, l'invention concerne un dispositif du type précité, selon la revendication 1.

Dans le dispositif de l'invention, l'élément de blocage est donc formé par un élément filaire dont les branches sont destinées à prendre appui sur des zones du corps entourant l'articulation et non directement sur celle-ci. Dans le cas d'une orthèse de poignet, l'une des branches s'étend sur la paume de la main et l'autre sur le dos. Ainsi, l'élément filaire n'exerce pas de contrainte sur l'articulation, même en étant maintenu en place avec un certain serrage par le manchon et par la partie d'appui. En outre, la forme particulière de l'élément filaire permet de garantir son immobilité par rapport au reste du dispositif de maintien, garantissant ainsi l'immobilité de l'articulation lorsque le dispositif est porté.

Selon d'autres caractéristiques du dispositif de maintien de l'invention, il présente l'une ou plusieurs des caractéristiques des revendications 2 à 9.

D'autres aspects et avantages de l'invention apparaîtront à la lecture de la description qui suit, donnée à titre d'exemple et fait en références aux dessins annexés, dans lesquels :
- la Fig. 1 est une représentation schématique en perspective d'un dispositif de maintien selon l'invention formant une orthèse de poignet,
- la Fig. 2 est une représentation schématique en perspective, selon un angle de vue, d'un élément filaire selon un premier mode de réalisation de moyens de blocage utilisés avec un dispositif de maintien de la Fig. 1,
- la Fig. 3 est une représentation schématique en perspective, selon un autre angle de vue, de l'élément filaire de la Fig. 2,
- la Fig. 4 est une représentation schématique en perspective d'un élément filaire selon un deuxième mode de réalisation de moyens de blocage utilisés avec un dispositif de maintien de la Fig. 1,
- la Fig. 5 est une représentation schématique de côté d'un dispositif de maintien de la Fig. 1 porté par la main d'un utilisateur,
- la Fig. 6 est une représentation schématique de côté de l'application d'un élément filaire des Fig. 2 et 3 sur la main d'un utilisateur lorsqu'il porte un dispositif de maintien selon l'invention, et
- la Fig. 7 est une représentation schématique de l'autre côté de l'application d'un élément filaire des Fig. 2 et 3 sur la main d'un utilisateur lorsqu'il porte un dispositif de maintien selon l'invention.

La description sera faite en référence à un dispositif de maintien 1, plus particulièrement un dispositif médical formant une orthèse de poignet, par exemple adaptée pour le traitement de la rhizarthrose. Il est cependant entendu que le dispositif médical pourrait être adapté pour d'autres parties du corps d'un utilisateur, ou patient, afin de maintenir une articulation dans une position particulière. Ainsi le dispositif médical selon l'invention pourrait être adapté pour former une genouillère, une coudière, une chevillère ou une ceinture par exemple. De façon générale, le dispositif médical selon l'invention peut être adapté pour former une attelle, une ceinture, un collier, un support, un vêtement compressif, un article tissé ou tricoté, ou autre article similaire, utilisé pour la thérapie ou le maintien d'une partie du corps. Le dispositif de maintien peut également être utilisé en dehors du domaine médical. En effet, le dispositif peut par exemple être utilisé dans le domaine sportif, par exemple pour former une chaussure pour la pratique du basket-ball ou un chausson de planche à voile, ou tout autre domaine nécessitant le maintien d'une articulation d'un utilisateur.

Dans le reste de la description, faite en référence à une orthèse de poignet, la première partie du corps du patient est formée par le pouce, la deuxième partie du corps du patient est formée par l'index et l'articulation est formée par l'articulation trapézo-métacarpienne de la main, c'est-à-dire une articulation entre le pouce et l'index.

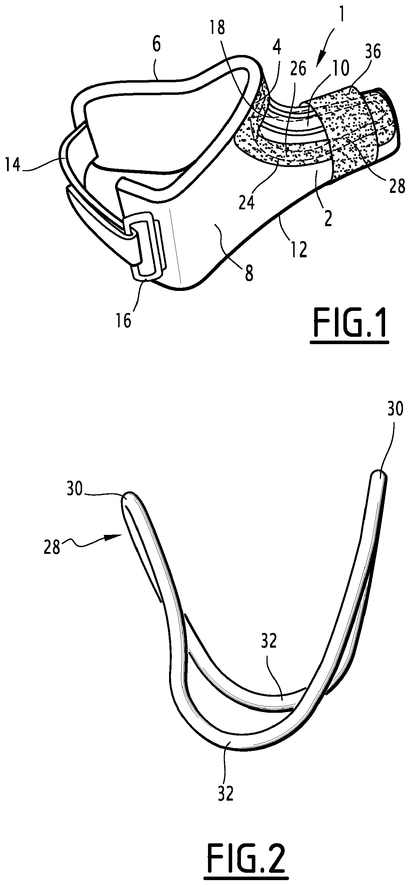

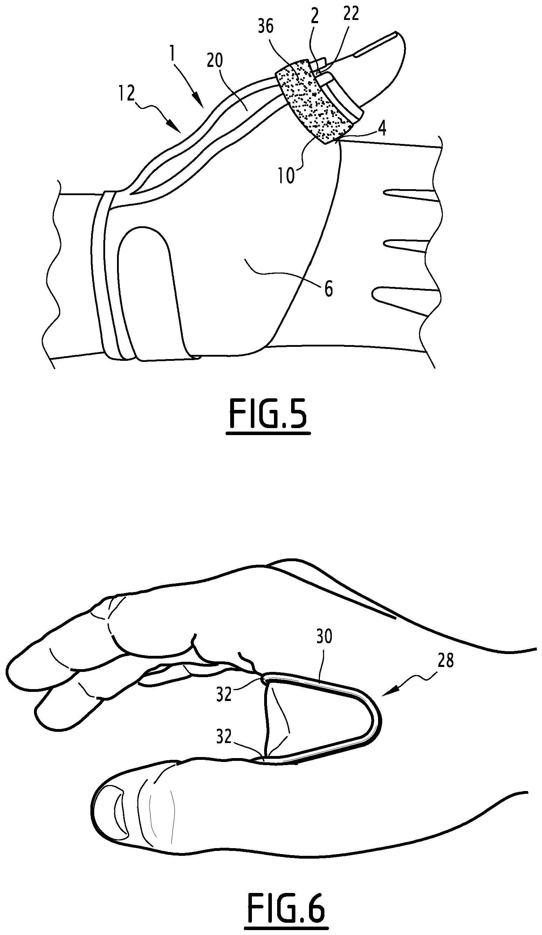

En référence à la Fig. 1, on décrit un dispositif médical 1, formant une orthèse de poignet, comprenant un manchon 2 et une partie d'appui 4 reliant l'une à l'autre une première face 6 et une deuxième face 8 du dispositif 1. La première face 6 forme la face palmaire de l'orthèse, c'est-à-dire qu'elle est destinée à recouvrir au moins une partie de la paume de la main lorsque l'orthèse est portée par un patient comme cela est représenté sur la Fig. 5, et la deuxième face 8 forme la face dorsale de l'orthèse, c'est-à-dire qu'elle est destinée à recouvrir au moins une partie du dos de la main lorsque l'orthèse est portée par un patient. Le manchon 2 est destiné à entourer au moins une partie du pouce de la main et la partie d'appui 4 est destinée à être en appui contre la base de l'index de la main lorsque l'orthèse est portée par un patient, comme représenté sur la Fig. 5. La première face 6 et la deuxième face 8 sont reliées l'une à l'autre au niveau d'une zone de jonction 10 s'étendant entre le manchon 2 et la partie d'appui 4 et destinée à recouvrir la première commissure de la main et au niveau d'une zone latérale 12 s'étendant dans la continuité du manchon 2 et destinée à recouvrir une partie de la colonne du pouce et une partie du poignet lorsque l'orthèse est portée par un patient comme représenté sur la Fig. 5.

Le manchon 2, la partie d'appui 4, la première face 6 et la deuxième face 8 sont réalisés d'une seule pièce ou de plusieurs pièces assemblées entre elles, par couture, soudure ou collage, en matériau textile ou matériau souple de sorte à définir une enveloppe 12 recouvrant une partie de la main lorsque l'orthèse est portée. A titre d'exemple, le matériau textile est un complexe textile tridimensionnel respirant, constitué pour la partie intérieure, c'est-à-dire destinée à être en contact avec la main, d'un tricot tridimensionnel en polyester et pour la partie extérieure d'une maille grattée en polyamide. Le matériau est donc adapté au port de l'orthèse par un patient de façon confortable, même pour des périodes prolongées.

L'enveloppe 12 comprend, de façon classique, à sa partie extrême opposée au manchon 2 et à la partie d'appui 4, destinée à s'étendre autour du poignet de la main, une sangle 14 de réglage permettant de rapprocher les parties extrêmes de la première face 6 et de la deuxième face 8 l'une de l'autre afin de régler le serrage de l'orthèse autour du poignet. La sangle 14 est par exemple cousue, soudée ou collée à l'une des parties extrêmes, passe par un passant 16 cousu, soudé ou collé à l'autre partie extrême et est rabattue sur elle-même de sorte à être fixée sur elle-même ou sur l'enveloppe 12 par des moyens autoagrippants, comme représenté sur la Fig. 1. Il est entendu que les moyens autoagrippants peuvent être remplacés par tout autre système d'accroche rapide adapté, tel que des aimants, des boutons pression ou autres.

Selon le mode de réalisation représenté sur les Fig. 1 et 5, l'enveloppe 12 comprend une fente 18 au niveau de la zone de jonction 10. Un matériau textile différent du matériau textile formant l'enveloppe s'étend dans la fente 18. Ce matériau textile est par exemple cousu, soudé ou collé à l'enveloppe, le long des bords de la fente 18. Ce matériau textile est agencé pour ne pas exercer de contrainte sur la première commissure qu'il recouvre. Un tel matériau est par exemple un textile tricoté tridimensionnel, offrant respirabilité et élasticité. Ainsi, on s'assure qu'aucune contrainte n'est exercée contre la première commissure qui peut être une zone douloureuse traitée par l'orthèse.

Selon le mode de réalisation représenté sur la Fig. 5, le manchon 2 et la zone latérale 12 comprennent une fente 20. Un matériau textile différent du matériau textile formant l'enveloppe s'étend dans la fente 20. Ce matériau textile est par exemple cousu, soudé ou collé à l'enveloppe, le long des bords de la fente 20. Ce matériau textile est agencé pour ne pas exercer de contrainte sur la colonne du pouce qu'il recouvre. Un tel matériau est par exemple un textile tricoté comprenant des fibres de polyester comportant du dioxyde de zirconium, tel que le Zirtex®. De la matière laminée ou enduite avec un film aluminisé ou céramique, des fibres céramiques, des fibres contenant du dioxyde de titane et/ou des fibres de polyacrylate sont également envisageables pour réaliser le textile s'étendant dans la fente 20. Ainsi, on s'assure qu'aucune contrainte n'est exercée contre la colonne du pouce qui est une zone douloureuse traitée par l'orthèse, notamment en cas de rhizarthrose. Dans le cas d'une fente 20 débouchant au niveau du manchon 2, le matériau textile ferme la fente 20 par une couture 22 solidarisant les deux extrémités de la fente 20.

Un gousset 24 est formé autour de la zone de jonction 10 entre le manchon 2 et la zone d'appui 4 de sorte à entourer la première commissure lorsque l'orthèse est portée par la main d'un patient. Le gousset 24 est par exemple formé entre une bande de matériau textile 26 fixée sur l'enveloppe 12 autour d'au moins une partie de la zone de jonction 10, c'est-à-dire autour de la fente 18, et l'enveloppe 12. Cette bande 26 s'étend sur une partie du manchon 2 en regard de la partie d'appui 4, sur la première face 6 au bord de la zone de jonction 10, sur la partie d'appui 4 en regard du manchon 2 et sur la deuxième face 8 au bord de la zone de jonction 10, de sorte que le gousset 24 présente une forme annulaire entourant la zone de jonction 10. La bande 26 est par exemple réalisée en un matériau doux, agencé pour ne pas irriter le pouce ou l'index en cas de frottement contre la bande 26. Ce matériau est par exemple constitué d'une maille grattée en polyamide.

Le gousset 24 forme un logement de réception de moyens de blocage de la fermeture de la première commissure. Ces moyens de blocage relient le manchon 2 à la partie d'appui 4. Les moyens de blocage sont formés par un élément filaire 28, par exemple en matériau métallique, agencé pour maintenir l'écart entre le manchon 2 et la partie d'appui 4 et empêcher la fermeture de la première commissure lorsque l'orthèse est portée par la main d'un patient. L'élément filaire 28 est agencé pour entourer la zone de jonction 10, sous la bande 26 formant le gousset 24 avec l'enveloppe 12 et présente une rigidité adaptée pour empêcher un mouvement du pouce vers l'index tendant à fermer la première commissure. L'élément filaire 28 maintient donc l'écart entre le manchon 2 et la partie d'appui 4. Le matériau choisi pour réaliser l'élément filaire 28 est léger tout en présentant la rigidité nécessaire pour remplir sa fonction. A cet effet, l'élément filaire 28 est par exemple réalisé à partir d'un matériau métallique du type acier, inox ou aluminium, ou à partir d'un matériau plastique du type polymère.

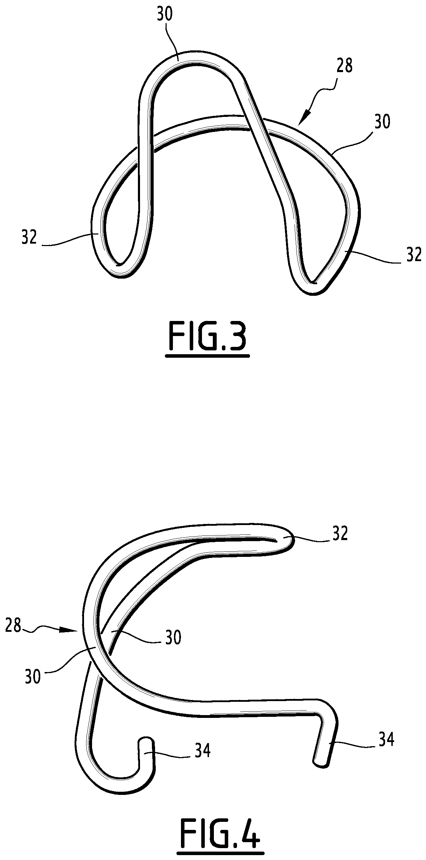

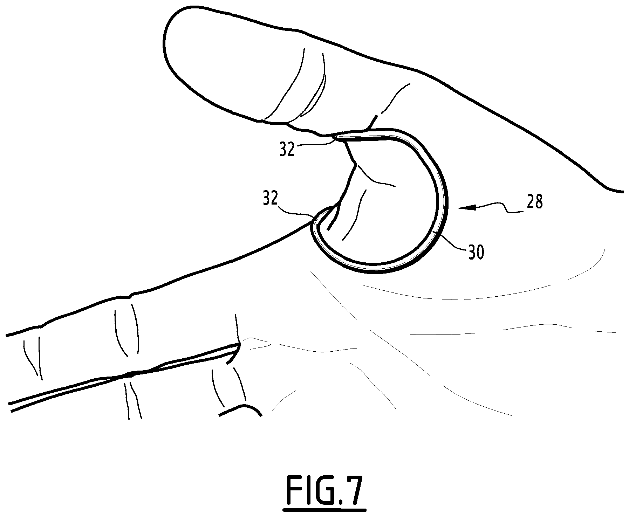

Selon le mode de réalisation représenté sur les Fig. 2 et 3, l'élément filaire 28 forme un anneau fermé suivant sensiblement le contour de la zone de jonction 10. A cet effet, l'élément filaire 28 comprend deux branches 30 reliées l'une à l'autre à leurs extrémités par deux parties de jonction 32. Les branches 30 sont donc espacées l'une de l'autre entre les parties de jonction 32, une des branches 30 passant sur la première face 6, le long de la zone de jonction 10 du manchon 2 à la partie d'appui 4, et l'autre branche 30 passant sur la deuxième face 8, le long de la zone de jonction 10 du manchon 2 à la partie d'appui 4, de sorte à entourer la première commissure lorsque l'orthèse est portée. Une partie de jonction 32 passe sur la partie du manchon 2 en regard de la partie d'appui 4, c'est-à-dire sur le pouce lorsque l'orthèse est portée, et l'autre partie de jonction 32 passe sur la partie d'appui 4 en regard du manchon 2, c'est-à-dire sur la base de l'index lorsque l'orthèse est portée, comme représenté schématiquement sur les Fig. 6 et 7.

Chaque branche 30 présente une forme courbe, par exemple une forme de U, le rayon de courbure de chaque branche 30 étant agencé pour que l'index et le pouce forme entre eux un angle fixe sensiblement compris entre 45° et 60°, correspondant à une ouverture naturelle de la première commissure.

Selon le mode de réalisation représenté sur les Fig. 2, 3, 6 et 7, chaque branche 30 présente une forme adaptée à la face de la main sur laquelle elle s'étend. Ainsi, la branche 30 s'étendant sur la première face 6, ou face palmaire, présente une forme s'approchant d'un arc de cercle s'étendant sous la base du pouce et sous la bas de l'index (Fig. 7), tandis que la branche 30 s'étendant sur la deuxième face 8, ou face dorsale, présente plutôt une forme allongée s'approchant d'une partie d'ellipse ou d'ovale, s'étendant jusqu'à la jonction entre le pouce et l'index, par exemple jusqu'à la base des métacarpiens (Fig. 6).

Selon un autre mode de réalisation, les deux branches 30 présentent une forme sensiblement identique.

On notera que du fait de la nature filaire de l'élément 28, la courbure des branches 30 peut être facilement adaptée afin de régler l'angle entre l'index et le pouce par simple déformation de l'élément filaire 28. Ainsi, les moyens de blocage peuvent être adaptés facilement à toutes les morphologies de main. Les parties de jonction 32 présentent également une forme adaptée à la morphologie de la main. Ainsi, les parties de jonction 32 peuvent être légèrement incurvées afin de s'adapter à la forme de la base de l'index et du pouce.

Plus précisément, l'élément filaire 28 vient en appui contre la tête du premier métacarpien du côté du pouce et contre la tête du deuxième métacarpien du côté de l'index.

Selon un deuxième mode de réalisation, représenté sur la Fig. 4, l'élément filaire 28 n'est pas fermé. L'élément filaire 28 selon ce mode de réalisation se distingue de celui décrit en référence à la Fig. 2 en ce qu'il ne comprend qu'une partie de jonction 32, par exemple celle passant sur le manchon 2. A l'autre extrémité des branches 30, l'élément filaire 28 est interrompu et comprend simplement un décrochement 34 s'étendant à partir de l'extrémité de chaque branche 30 sur la partie d'appui 4. L'élément filaire 28 selon ce mode de réalisation présente donc un profil ouvert.

L'orthèse comprend en outre une sangle 36 s'étendant autour du manchon 2. Cette sangle 36 est agencée pour permettre le réglage du serrage du manchon 2 autour du pouce, par exemple en rapprochant les bords de la fente 20 l'un de l'autre, et pour améliorer le maintien de l'élément filaire 28 dans le gousset 24. En effet, la sangle 36 est agencée pour passer au-dessus du gousset 24 en recouvrant une partie de l'élément filaire 28, afin de plaquer celui-ci contre le manchon 2. La sangle 36 est par exemple pourvue de moyens autoagrippants permettant de fixer la sangle 36 sur l'enveloppe 12 ou sur elle-même, après l'avoir passée autour du manchon 2.

L'orthèse décrite ci-dessus est particulièrement confortable et indolore à porter car les moyens de blocage n'exercent aucune pression directe sur la première commissure. En outre, les zones douloureuses de la main se trouvent en regard d'un matériau textile adapté pour n'exercer aucune contrainte sur ces zones, par l'intermédiaire des fentes 18 et 20. Les moyens de blocage n'exercent aucune contrainte contre la colonne du pouce, qui est une zone douloureuse en cas de rhizarthrose. En outre, le fait de prévoir des moyens de blocage sous forme d'un élément filaire 28 léger, permet d'alléger l'orthèse dans son ensemble et de permettre à celle-ci de présenter une bonne « respirabilité », les moyens de blocage ne recouvrant pas une grande zone de la main et occupant peu d'espace. L'orthèse est facile à régler et peut s'adapter à toutes les morphologies, du fait des sangles 14 et 36 et de l'élasticité relative des matériaux textiles disposés dans les fentes 18 et 20. L'orthèse est agencée pour permettre une mobilité des doigts en dehors du blocage de la fermeture de la première commissure, ainsi cette orthèse n'empêche pas outre mesure le patient de se servir de sa main lorsqu'il porte l'orthèse.

Comme indiqué précédemment, bien que le dispositif de maintien selon l'invention ait été décrit en relation avec une orthèse de poignet, il est entendu qu'il peut être utilisé pour d'autres zones du corps, en adaptant simplement les dimensions des différents éléments décrits précédemment, aussi bien dans le domaine médical que dans d'autres domaines.

## Revendications

1. Dispositif de maintien (1) d'une articulation d'un utilisateur reliant une première partie du corps dudit utilisateur à une deuxième partie du corps dudit utilisateur, comprenant une première face (6) et une deuxième face (8) reliées l'une à l'autre par au moins un manchon (2), destiné à entourer la première partie du corps de l'utilisateur, et par une partie d'appui (4), destinée à être en appui contre la deuxième partie du corps dudit utilisateur, ledit manchon (2) et ladite partie d'appui (4) étant reliés l'un à l'autre par des moyens de blocage de la fermeture de l'articulation,
lesdits moyens de blocage comprenant un élément filaire (28) comprenant au moins deux branches (30), dont l'une s'étend du manchon (2) à la partie d'appui (4) sur la première face (6) du dispositif et l'autre s'étend du manchon (2) à la partie d'appui (4) sur la deuxième face (8) du dispositif, lesdites branches (30) étant espacées l'une de l'autre entre le manchon (2) et la partie d'appui (4) et étant agencées pour maintenir l'écart entre le manchon (2) et la partie d'appui (4) de part et d'autre de l'articulation, chaque branche (30) de l'élément filaire (28) présentant une forme courbe et étant reliée à l'autre branche par au moins une partie de jonction (32) s'étendant sur le manchon (2) et/ou sur la partie d'appui (4), de sorte que l'élément filaire (28) entoure la zone d'articulation sans exercer d'appui sur celle-ci,
le rayon de courbure de chaque branche (30) étant agencé pour que la première partie du corps et la deuxième partie du corps forment entre elles un angle fixe sensiblement compris entre 45° et 60°.

2. Dispositif de maintien selon la revendication 1, dans lequel l'élément filaire (28) est disposé dans un gousset (24) reliant le manchon (2) à la partie d'appui (4).

3. Dispositif de maintien selon l'une quelconque des revendications 1 à 2, dans lequel l'élément filaire (28) comprend deux parties de jonction (32) reliant les branches (30), l'une s'étendant sur le manchon (2) et l'autre s'étendant sur la partie d'appui (4).

4. Dispositif de maintien selon la revendication 3, dans lequel l'élément filaire (28) forme un anneau fermé.

5. Dispositif de maintien selon l'une quelconque des revendications 1 à 4, dans lequel le manchon (2) comprend au moins une fente (18, 20), les bords de ladite fente (18, 20) étant reliés l'un à l'autre par un matériau textile tricoté agencé pour ne pas appliquer d'appui contre la partie du corps recouverte par ledit matériau.

6. Dispositif de maintien selon la revendication 5, dans lequel le matériau textile tricoté comprend des fibres de polyester comprenant du dioxyde de zirconium.

7. Dispositif de maintien selon l'une quelconque des revendications 1 à 6, comprenant une sangle (36) s'étendant autour du manchon (2), ladite sangle (36) étant agencée pour permettre le réglage du serrage dudit manchon (2) autour de la première partie du corps.

8. Dispositif de maintien selon la revendication 7, dans lequel la sangle (36) recouvre une partie de l'élément filaire (28).

9. Dispositif de maintien selon l'une quelconque des revendications 1 à 8, configuré de sorte à former une orthèse de poignet, la première face (6) du dispositif étant destinée à s'étendre sur la paume de la main de l'utilisateur, la deuxième face (8) étant destinée à s'étendre sur le dos de la main, le manchon (2) étant destiné à entourer au moins une partie du pouce de la main et la partie d'appui (4) étant destinée à être en appui contre la base de l'index de ladite main, les moyens de blocage étant agencés pour empêcher la fermeture de la première commissure de la main.

## Patentansprüche

1. Vorrichtung zum Halten (1) eines Gelenks eines Benutzers, welches einen ersten Teil des Körpers des Benutzers mit einem zweiten Teil des Körpers des Benutzers verbindet, aufweisend eine erste Fläche (6) und eine zweite Fläche (8), die durch mindestens eine Hülse (2), die vorgesehen ist, um den ersten Teil des Körpers des Benutzers zu umgeben, und durch einen Stützteil (4), der vorgesehen ist, um an dem zweiten Teil des Körpers des Benutzers abgestützt zu sein, miteinander verbunden sind, wobei die Hülse (2) und der Stützteil (4) durch Mittel zum Blockieren des Schließens des Gelenks miteinander verbunden sind,
wobei die Mittel zum Blockieren ein Drahtelement (28) aufweisen, das mindestens zwei Zweige (30) aufweist, von denen sich der eine an der ersten Fläche (6) der Vorrichtung von der Hülse (2) bis zum Stützteil (4) erstreckt und sich der andere an der zweiten Fläche (8) der Vorrichtung von der Hülse (2) bis zum Stützteil (4) erstreckt, wobei die Zweige (30) zwischen der Hülse (2) und dem Stützteil (4) im Abstand voneinander sind und eingerichtet sind, um den Abstand zwischen der Hülse (2) und dem Stützteil (4) auf beiden Seiten des Gelenks aufrechtzuerhalten, wobei jeder Zweig (30) des Drahtelements (28) eine gekrümmte Form aufweist und durch mindestens einen Verbindungsteil (32) mit dem anderen Zweig verbunden ist, der sich an der Hülse (2) und/oder an dem Stützteil (4) erstreckt, so dass das Drahtelement (28) den Gelenkbereich umgibt, ohne Belastung auf diesen auszuüben,
wobei der Krümmungsradius jedes Zweiges (30) eingerichtet ist, damit der erste Teil des Körpers und der zweite Teil des Körpers zwischen einander einen festen Winkel ausbilden, der im Wesentlichen zwischen 45° und 60° liegt.

2. Vorrichtung zum Halten gemäß Anspruch 1, wobei das Drahtelement (28) in einem Achselstück (24) angeordnet ist, welches die Hülse (2) mit dem Stützteil (4) verbindet.

3. Vorrichtung zum Halten gemäß irgendeinem der Ansprüche 1 bis 2, wobei das Drahtelement (28) zwei Verbindungsteile (32) aufweist, die die Zweige (30) miteinander verbinden, wobei sich der eine an der Hülse (2) erstreckt und sich der andere an dem Stützteil (4) erstreckt.

4. Vorrichtung zum Halten gemäß Anspruch 3, wobei das Drahtelement (28) einen geschlossenen Ring ausbildet.

5. Vorrichtung zum Halten gemäß irgendeinem der Ansprüche 1 bis 4, wobei die Hülse (2) mindestens einen Schlitz (18, 20) aufweist, wobei die Ränder des Schlitzes (18, 20) durch ein Stricktextilmaterial miteinander verbunden sind, welches eingerichtet ist, um keine Belastung auf den Teil des Körpers auszuüben, der von dem Material bedeckt ist.

6. Vorrichtung zum Halten gemäß Anspruch 5, wobei das Stricktextilmaterial Polyesterfasern aufweist, die Zirconiumdioxid aufweisen.

7. Vorrichtung zum Halten gemäß irgendeinem der Ansprüche 1 bis 6, aufweisend ein Band (36), welches sich um die Hülse (2) herum erstreckt, wobei das Band (36) eingerichtet ist, um das Einstellen des Einspannens der Hülse (2) um den ersten Teil des Körpers zu ermöglichen.

8. Vorrichtung zum Halten gemäß Anspruch 7, wobei das Band (36) einen Teil des Drahtelements (28) bedeckt.

9. Vorrichtung zum Halten gemäß irgendeinem der Ansprüche 1 bis 8, die derart konfiguriert ist, dass sie eine Handgelenksorthese ausbildet, wobei die erste Fläche (6) der Vorrichtung vorgesehen ist, um sich an der Handfläche der Hand des Benutzers zu erstrecken, wobei die zweite Fläche (8) vorgesehen ist, um sich am Handrücken zu erstrecken, wobei die Hülse (2) vorgesehen ist, um mindestens einen Teil des Daumens der Hand zu umgeben, und der Stützteil (4) vorgesehen ist, um an der Basis des Zeigefingers der Hand abgestützt zu sein, wobei die Mittel zum Blockieren eingerichtet sind, um das Schließen der ersten Kommissur der Hand zu verhindern.

## Claims

1. Support device (1) for a joint of a user connecting a first part of the body of said user to a second part of the body of said user, which device comprises a first face (6) and a second face (8) which are connected to one another by at least one sleeve (2), which is to surround the first part of the body of the user, and by a bearing part (4), which is to bear against the second part of the body of said user, said sleeve (2) and said bearing part (4) being connected to one another by means for blocking closure of the joint,
said blocking means comprising a wire element (28) comprising at least two branches (30), of which one extends from the sleeve (2) to the bearing part (4) on the first face (6) of the device and the other extends from the sleeve (2) to the bearing part (4) on the second face (8) of the device, said branches (30) being spaced apart from one another between the sleeve (2) and the bearing part (4) and being arranged to maintain the spacing between the sleeve (2) and the bearing part (4) on either side of the joint, each branch (30) of the wire element (28) having a curved shape and being connected to the other branch by at least one joining part (32) which extends on the sleeve (2) and/or on the bearing part (4) so that the wire element (28) surrounds the joint zone without exerting pressure thereon,
the radius of curvature of each branch (30) being such that the first part of the body and the second part of the body form between them a fixed angle of substantially between 45° and 60°.

2. Support device according to claim 1, wherein the wire element (28) is arranged in a gusset (24) which connects the sleeve (2) to the bearing part (4).

3. Support device according to either claim 1 or claim 2, wherein the wire element (28) comprises two joining parts (32) connecting the branches (30), one extending on the sleeve (2) and the other extending on the bearing part (4).

4. Support device according to claim 3, wherein the wire element (28) forms a closed ring.

5. Support device according to any one of claims 1 to 4, wherein the sleeve (2) comprises at least one slot (18, 20), the edges of said slot (18, 20) being connected to one another by a knitted textile material which is designed not to apply pressure to the part of the body that is covered by said material.

6. Support device according to claim 5, wherein the knitted textile material comprises polyester fibres comprising zirconium dioxide.

7. Support device according to any one of claims 1 to 6, comprising a strap (36) which extends around the sleeve (2), said strap (36) being arranged to allow the tightness of said sleeve (2) around the first part of the body to be adjusted.

8. Support device according to claim 7, wherein the strap (36) covers part of the wire element (28).

9. Support device according to any one of claims 1 to 8, configured so as to form a wrist orthosis, the first face (6) of the device being intended to extend over the palm of the hand of the user, the second face (8) being intended to extend over the back of the hand, the sleeve (2) being intended to surround at least part of the thumb of the hand, and the bearing part (4) being intended to bear against the base of the index finger of said hand, the blocking means being arranged to prevent closure of the first commissure of the hand.
